(19) Europäisches Patentamt / European Patent Office / Office européen des brevets

(11) **EP 3 918 990 A1**

(12) **EUROPEAN PATENT APPLICATION**
published in accordance with Art. 153(4) EPC

(43) Date of publication:
**08.12.2021 Bulletin 2021/49**

(51) Int Cl.:
***A61B 5/11*** (2006.01)

(21) Application number: **19912571.7**

(86) International application number:
**PCT/JP2019/002798**

(22) Date of filing: **28.01.2019**

(87) International publication number:
**WO 2020/157808 (06.08.2020 Gazette 2020/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **FUJITSU LIMITED**
**Kanagawa 211-8588 (JP)**

(72) Inventors:
• **HOTTA, Shinji**
**Kawasaki-shi, Kanagawa 211-8588 (JP)**
• **KOMABA, Yusuke**
**Kawasaki-shi, Kanagawa 211-8588 (JP)**
• **YAGINUMA, Yoshinori**
**Kawasaki-shi, Kanagawa 211-8588 (JP)**

(74) Representative: **Hoffmann Eitle**
**Patent- und Rechtsanwälte PartmbB**
**Arabellastraße 30**
**81925 München (DE)**

(54) **SENSOR ATTACHMENT/DETACHMENT IDENTIFICATION PROGRAM, SYSTEM, AND METHOD**

(57) A sensor attachment/detachment determination program comprising instructions which, when the program is executed by a computer, cause the computer to execute processing, the processing including: extracting a frequency characteristic of time-series data acquired from a gyro sensor; and determining a sensor attachment/detachment period according to a section in which data indicating a high-frequency characteristic in which a frequency is equal to or higher than a predetermined value is continuously generated, among the time-series data, on the basis of the frequency characteristic.

## FIG. 3

EP 3 918 990 A1

**Description**

TECHNICAL FIELD

The disclosed techniques relate to a sensor attachment/detachment determination program, a sensor attachment/detachment determination system, and a sensor attachment/detachment determination method.

BACKGROUND ART

In recent years, it is needed to discharge patients early and take care of the patients at home due to a shortage of doctors and beds in hospitals. Therefore, there is a need for a mechanism that automatically measures a patient's recovery state from illness at home and allows medical staff to grasp measurement results from a remote location such as a hospital. For example, gait features are used in clinical sites as an indicator of the recovery state. Information of the gait features and the like is obtained by attaching a sensor such as a gyro sensor to the patient, acquiring time-series data, and analyzing the acquired data.

For example, a stair walk recognition device that identifies a subject's stair walk from a flat ground walk has been proposed. The device includes a triaxial acceleration sensor and a triaxial vibration gyro that monitor a subject's body movement. Since the triaxial vibration gyro detects a rotational angular velocity of a subject's foot in a front-rear direction, the stair walk is identified on the basis of data thereof.

Furthermore, a gait analysis system has been proposed, which is provided with a measurement sensor that is attached to walker's both feet so as to sandwich a hip joint, a knee joint, and an ankle joint, measures an angular acceleration and an acceleration during walk of the walker, and outputs measurement data, and a gait analysis unit. In this system, the gait analysis unit evaluates the gait state on the basis of the measurement data.

Furthermore, a technique for appropriately correcting a gyro sensor that detects a walker's movement has been proposed. This technique corrects angular velocity information acquired by the gyro sensor according to determination that a user is in a straight-ahead state on the basis of a correlation among acceleration information, angular acceleration information, and image information.

Furthermore, a technique for detecting peaks from time-series data acquired from a triaxial gyro sensor, extracting a swing motion candidate, and performing correction by multiplying the time-series data by a rotation matrix so as to maximize a forward rotation speed of the swing motion candidate, and then extracting a gait feature has been proposed.

SUMMARY OF INVENTION

TECHNICAL PROBLEM

However, in the prior art, data at the time of attachment/detachment of the sensor to/from the subject is not considered. When analyzing the time-series data acquired by the gyro sensor, if the time-series data includes the data at the time of attaching/detaching the gyro sensor to/from the subject, there is a problem that an appropriate analysis result is not able to be obtained.

As one aspect, the disclosed technique aims to accurately determine data at the time of attachment/detachment of a sensor, which is not data to be analyzed.

SOLUTION TO PROBLEM

In one embodiment, the disclosed technique extracts frequency characteristics of time-series data acquired from a gyro sensor. Furthermore, the disclosed technique determines a sensor attachment/detachment period according to a section in which data indicating a high-frequency characteristic in which the frequency is equal to or higher than a predetermined value is continuously generated, among the time-series data, on the basis of the frequency characteristics.

ADVANTAGEOUS EFFECTS OF INVENTION

As one aspect, an effect of accurately determining data at the time of attachment/detachment of a sensor, which is not data to be analyzed, is obtained.

BRIEF DESCRIPTION OF DRAWINGS

FIG. 1 is a diagram illustrating an example of gyro data;

FIG. 2 is a functional block diagram of a sensor attachment/detachment determination system;

FIG. 3 is a diagram for describing extraction of frequency characteristics;

FIG. 4 is a diagram for describing threshold value determination of the frequency characteristics;

FIG. 5 is a diagram for describing determination of an attachment/detachment period;

FIG. 6 is a diagram for describing determination of the attachment/detachment period;

FIG. 7 is a diagram for describing mount deviation of the gyro sensor;

FIG. 8 is a diagram for describing rotation correction;

FIG. 9 is a diagram for describing detection of a walk section;

FIG. 10 is a diagram illustrating an example of a table in which condition determination elements are recorded;

FIG. 11 is a block diagram illustrating a schematic configuration of a computer that functions as the sensor attachment/detachment determination system;

FIG. 12 is a flowchart illustrating an example of sensor attachment/detachment determination processing;

FIG. 13 is a flowchart illustrating an example of attachment/detachment determination processing in a first embodiment;

FIG. 14 is a flowchart illustrating an example of analysis processing;

FIG. 15 is a diagram for describing removal of the determined attachment/detachment period;

FIG. 16 is a diagram schematically illustrating an example of gyro data when sensors are alternately mounted;

FIG. 17 is a diagram schematically illustrating an example of gyro data when sensors are mounted at the same time;

FIG. 18 is a diagram for describing calculation of a right-left difference in differential values of the gyro data;

FIG. 19 is a diagram for describing calculation of a signed right-left difference in differential values of the gyro data;

FIG. 20 is a diagram for describing calculation of a right-left product in differential values of the gyro data;

FIG. 21 is a diagram for describing threshold value determination of a right-left difference;

FIG. 22 is a diagram for describing threshold value determination of a signed right-left difference;

FIG. 23 is a diagram for describing threshold value determination of a right-left product;

FIG. 24 is a diagram for describing a margin to be added to the attachment/detachment period determined on the basis of a segment in which the right-left product exceeds the threshold value; and

FIG. 25 is a flowchart illustrating an example of attachment/detachment determination processing in a second embodiment.

DESCRIPTION OF EMBODIMENTS

Hereinafter, an example of an embodiment according to the disclosed technology will be described with reference to the drawings.

First, data at the time of attachment/detachment of a sensor and data to be analyzed will be described before details of each embodiment is described. Note that, in each of the following embodiments, a case of analyzing gait features of a subject on the basis of time-series data (hereinafter also referred to as "gyro data") acquired from gyro sensors attached to predetermined parts (for example, ankles or the like) of right and left feet of the subject will be described.

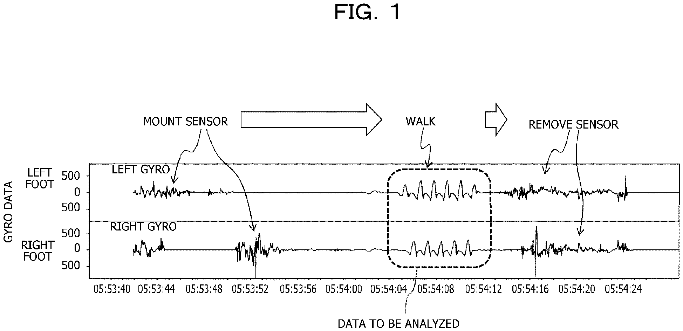

FIG. 1 illustrates an example of the gyro data acquired during a series of movements in which the subject wears the gyro sensors on both feet, walks, and removes the gyro sensors from both the feet. Note that, hereinafter, the gyro data acquired from the gyro sensor attached to the left foot will be referred to as left gyro data, and the gyro data acquired from the gyro sensor attached to the right foot will be referred to as right gyro data.

Here, data to be analyzed is data during walk, but to specify the data during walk, it is conceivable to extract data in a section in which a value changes by a predetermined value or more, for example. However, since the value similarly changes by a predetermined value or more at the time of attachment/detachment of the gyro sensor, there are some cases where data in this section is erroneously specified as the data during walk. In this case, an appropriate analysis result is not able to be obtained.

Therefore, in each of the following embodiments, the gait feature is analyzed after determining data at the time of attachment/detachment of the sensor in the gyro data, and excluding the data at the time of attachment/detachment of the sensor.

Hereinafter, each embodiment will be described in detail.

<First Embodiment

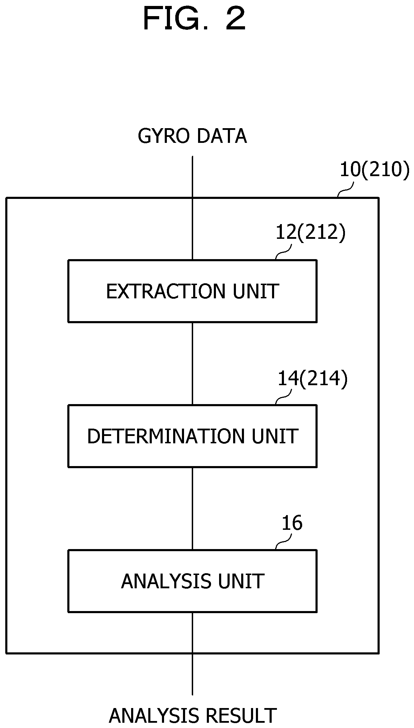

As illustrated in FIG. 2, gyro gyro data is input to a sensor attachment/detachment determination system 10. The gyro sensor is, for example, a triaxial gyro sensor that outputs gyro data in each of up-down, right-left, and front-rear directions with respect to a mount position. The sensor attachment/detachment determination system 10 performs sensor attachment/detachment determination and analysis processing for input gyro data and outputs an analysis result.

Functionally, as illustrated in FIG. 2, the sensor attachment/detachment determination system 10 includes an extraction unit 12, a determination unit 14, and an analysis unit 16.

The extraction unit 12 extracts frequency characteristics of the input gyro data. The characteristics of the gyro data at the time of attachment/detachment of the sensor include a high frequency of a waveform of the time-series data. This is because a change in a movement of the gyro sensor is faster at the time of attachment/detachment of the gyro sensor than a foot's movement during walk, or the like. Therefore, the extraction unit 12 extracts a frequency characteristic from which whether indicating a high-frequency characteristic in which the frequency is equal to or higher than a predetermined value can be determined, as a frequency characteristic.

For example, the extraction unit 12 extracts a differential value (d(t) = x(t) - x(t - 1)) of a value of each time of the gyro data as the frequency characteristic. Note that x(t) is the value of the gyro data at time t.

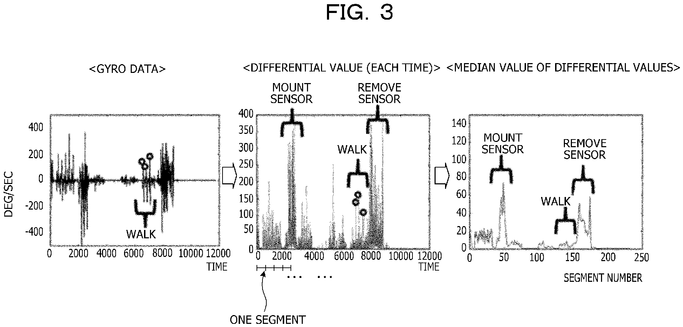

Here, as described above, in the present embodiment, a high-frequency characteristic is used as the characteristic of the gyro data at the time of attachment/detachment of the sensor. However, as illustrated by the circles in FIG. 3, in a case of obtaining the frequency characteristic (the differential value in this case) at each time, the data indicating a high-frequency characteristic is intermittently generated even in the case of data during walk. Meanwhile, at the time of attachment/detachment of the sensor, the data indicating a high-frequency characteristic is continuously generated, as compared with during walk.

Therefore, the extraction unit 12 extracts a frequency characteristic from which whether the data indicating a high-frequency characteristic is continuously generated can be determined. For example, the extraction unit 12 extracts a median value of the differential values included in a predetermined time unit as the frequency characteristic. Note that another statistical value such as an average value or a quantile may be used instead of the median value. Thereby, as illustrated in FIG. 3, the frequency characteristic from which the intermittent high frequency generated during walk is removed can be extracted.

Specifically, as illustrated in FIG. 3, the extraction unit 12 obtains, for each segment, the median value of the differential values in the segment, the each segment being obtained by dividing the differential values for respective times in the predetermined time unit (for example, two seconds). The extraction unit 12 assigns a segment number to each segment in chronological order, and passes an extraction result in which the median value obtained for that segment is associated with the segment number to the determination unit 14. Note that, hereinafter, a segment with a segment number i is described as "segment i".

The determination unit 14 determines a segment in which the median value of the differential values is equal to or larger than a predetermined threshold value TH1 as the section in which the data indicating a high-frequency characteristic is continuously generated on the basis of the extraction result passed from the extraction unit 12. Then, the determination unit 14 determines the sensor attachment/detachment period according to the section in which the data indicating a high-frequency characteristic is continuously generated.

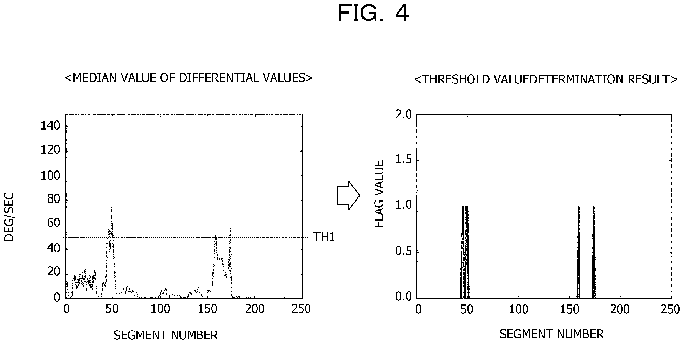

Specifically, as illustrated in FIG. 4, the determination unit 14 assigns a flag "1" to the segment in which the median value of the differential values is equal to or larger than the threshold value TH1, and assigns a flag "0" to a segment in which the median value of the differential values is less than the threshold value TH1, as a threshold value determination result.

Furthermore, in a case where an interval of successive sections among a plurality of sections in which the data indicating a high-frequency characteristic is continuously generated is equal to or smaller than a predetermined time, the determination unit 14 connects the successive sections. This is to avoid an intermittent attachment/detachment period in a case where, for example, the movement is stopped for a moment during attachment/detachment of the sensor if the attachment/detachment period is determined as it is on the basis of the threshold value determination result. As the predetermined time for determining whether to connect the sections, a time (for example, five seconds) is set in advance in consideration of the presence of time during which some movement such as walk is performed from when the sensor is attached to when the sensor is removed.

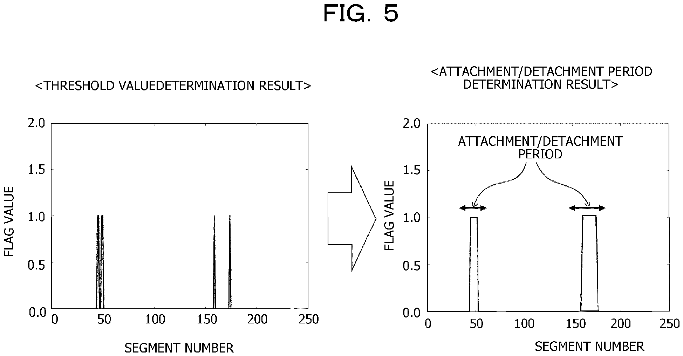

Specifically, as illustrated in FIG. 5, in a case where a time between successive segments in chronological order is equal to or less than a predetermined time, among the segments to which the flag "1" is assigned of the threshold value determination result, the determination unit 14 changes the flag of a segment between the segments to "1". For example, assuming that, in a case where the flag of a segment 1 is "1", the flag of a segment 2 is "0", and the flag of a segment 3 is "1", the time between the segment 1 and the segment 3 is four seconds, and the predetermined time is five seconds. In this case, the determination unit 14 connects the threshold value determination result of the segment 1 and the threshold value determination result of the segment 3 by setting the flag of the segment 2 to "1".

Furthermore, as illustrated in FIG. 5, the determination unit 14 determines a section obtained by adding a predetermined margin to areas before and after the sections of the connected threshold value determination results as the attachment/detachment period of the sensor. This is in consideration of a possibility that the sensor is attached/detached even before and after the segment in which the median value of the differential values of the gyro data exceeds the threshold value.

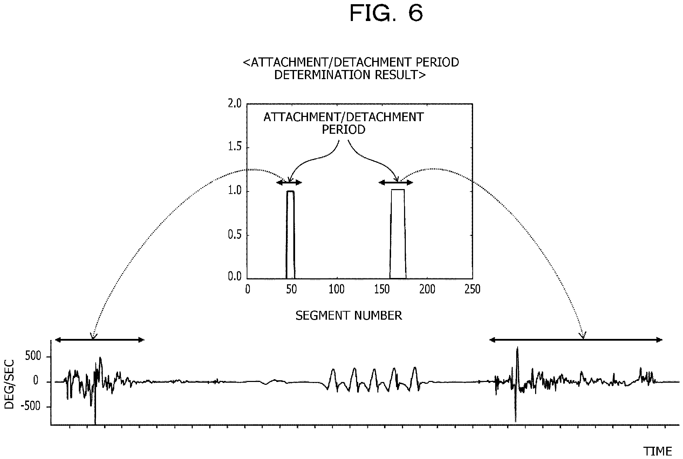

As illustrated in FIG. 6, the determination unit 14 specifies the attachment/detachment period in which the attachment/detachment period determined in the segment unit is caused to correspond to the time of the gyro data, which is the original time-series data, and passes information of the specified attachment/detachment period to the analysis unit 16.

The analysis unit 16 performs predetermined analysis processing, using the data of the sections obtained by excluding the data in the attachment/detachment period passed from the determination unit 14, of the input gyro data, as the data to be analyzed. In the present embodiment, a case of analyzing a gait feature will be described in detail as an example.

The analysis unit 16 sets while shifting a predetermined window width (for example, five seconds) for the data to be analyzed excluding the attachment/detachment period, and extracts a section having a change in the value of data in the window width as a walk section candidate. The analysis unit 16 performs rotation correction, detection of the walk section, and extraction of the gait feature for each walk section candidate. Hereinafter, each of the rotation correction, the detection of the walk section, and the extraction of the gait feature will be described.

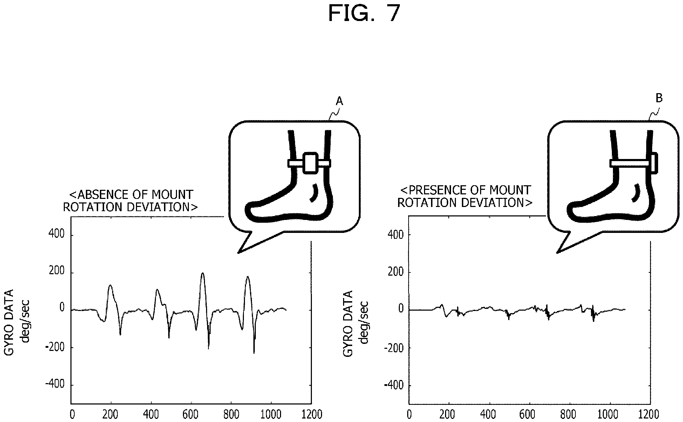

First, the rotation correction will be described. For example, in a case of acquiring the gyro data of the triaxial gyro sensor, it is assumed that the gyro sensor is mounted in a correct orientation in which the mount position of the gyro sensor does not have a rotation deviation, as illustrated in A of FIG. 7. In this case, the gait feature can be easily extracted from the gyro data in the front-rear direction. Meanwhile, in a case where the mount position of the gyro sensor has a rotation deviation, as illustrated in B of FIG. 7, appropriate gyro data is not able to be acquired, and extraction of the gait feature may become difficult. To solve this problem, the analysis unit 16 performs the rotation correction.

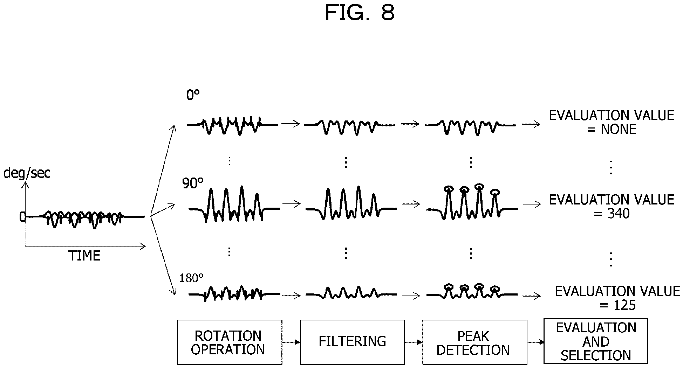

As illustrated in FIG. 8, the analysis unit 16 performs rotation operation, filtering, peak detection, evaluation and selection, as rotation correction of the gyro data.

Specifically, the analysis unit 16 applies a rotation matrix by performing the following calculation operations for a plurality of candidate angles using the gyro data in the front-rear direction and the gyro data in the right-left direction as the rotation operation and obtains a candidate gyroX'(t) for gyro data in the front-rear direction after the rotation operation.

$$\text{gyroX'(t)} = \text{gyroX(t)} \cdot \cos\theta - \text{gyroY(t)} \cdot \sin\theta$$

Here, gyroX(t) is the value of the gyro data in the front-rear direction at the time t, gyroY(t) is the value of the gyro data in the right-left direction at the time t, and θ is the candidate angle.

Furthermore, as filtering processing, the analysis unit 16 applies a low-pass filter to each candidate of the gyro data in the front-rear direction after the rotation operation to remove a minute noise peak before performing the peak detection.

Furthermore, as the peak detection, the analysis unit 16 detects a peak from each candidate of the filtered gyro data in the front-rear direction.

Furthermore, as evaluation and selection, the analysis unit 16 obtains, for example, an average of a predetermined number of upper amplitudes of the detected peaks as an evaluation value, and selects the gyro data in the front-rear direction after the rotation correction corresponding to the candidate angle having the maximum evaluation value, as data for extracting the gait feature. This is based on a principle that since a person swings his/her foot forward when walking, the amplitude of the gyro data in the front-rear direction is maximized if the gyro sensor is mounted at a correct angle. In the subsequent processing, the gyro data selected here is used.

Here, in the present embodiment, since the data in the attachment/detachment period of the sensor determined by the determination unit 14 is excluded, the peak of the gyro data generated at the time of attachment/detachment of the sensor is not detected in the peak detection for rotation correction. Therefore, in the evaluation and selection, erroneous evaluation and selection based on the peak of the gyro data generated at the time of attachment/detachment of the sensor can be avoided, and the rotation correction can be performed with accuracy. For example, in the technique of Patent Document 4, since an agile motion when attaching/detaching a sensor enters immediately before and after a walk swing motion, this attachment/detachment motion is erroneously determined as a swing motion candidate, and erroneous rotation conversion is performed. In the present embodiment, such a problem can be solved.

Next, the detection of the walk section will be described. The analysis unit 16 performs, as the detection of the walk section, peak range determination, condition determination element extraction, and walk condition determination.

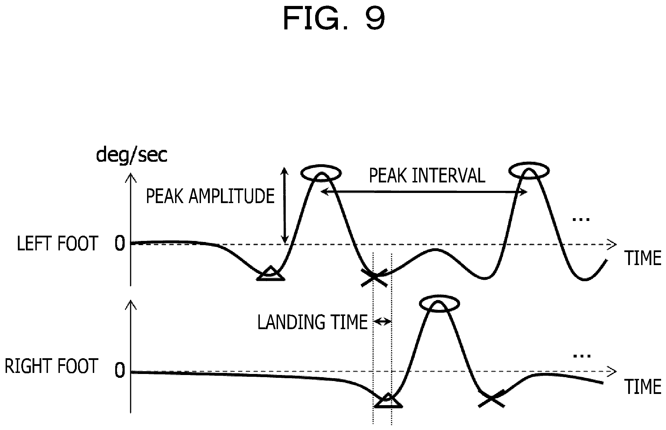

Specifically, the analysis unit 16 identifies a peak start point and a peak end point as the peak range determination. As illustrated in FIG. 9, the peak start point is a point where the gyro data value becomes the minimum (the triangular mark in FIG. 9) within a predetermined section before a peak maximum point (the elliptical mark in FIG. 9), and the peak end point is a point where the gyro data value becomes the minimum within a predetermined section after the peak maximum point (the cross mark in FIG. 9).

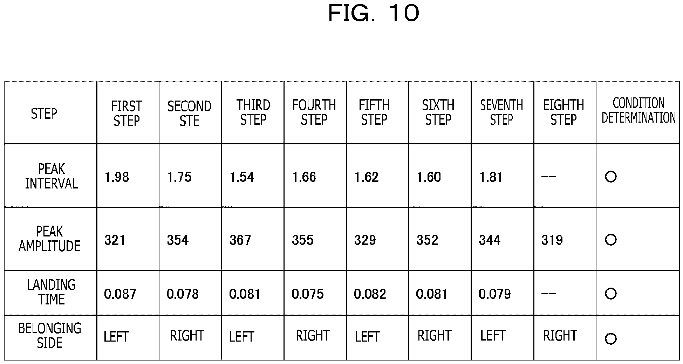

Furthermore, the analysis unit 16 assigns steps such as a first step, a second step, ..., for each peak that appears in the gyro data in chronological order, and extracts a peak interval, a peak amplitude, a landing time, and a foot belonging side for each step as the condition determination elements. As illustrated in FIG. 9, the peak interval is a time length from the peak maximum point to the next peak maximum point. The peak amplitude is a value of the gyro data at the peak maximum point. The landing time is a time length from the peak end point in the gyro data on one of the right and left sides to the next peak start point in the gyro data on another side. The foot belonging side is information indicating whether the gyro data to which the peak belongs is the left gyro data or the right gyro data. The analysis unit 16 records the extracted condition determination elements in a table as illustrated in FIG. 10, for example.

Furthermore, the analysis unit 16 determines whether each condition determination element satisfies a walk section condition as the walk condition determination. For example, the analysis unit 16 determines all of whether all the peak intervals fall within a predetermined range, whether all the peak amplitudes fall within a predetermined range, all the landing times are equal to or larger than 0, and all the peaks alternate right and left are satisfied. In a case where all the conditions are satisfied, the analysis unit 16 determines that the walk section candidate concerned satisfies the walk section condition and detects the walk section candidate as the walk section.

Furthermore, the analysis unit 16 extracts swing time, stride time, step length, and the like for each step included in the walk section, as the gait features. The stride time is a time length from the peak maximum point to the next peak maximum point. The swing time is a time length from the peak start point to the peak end point. The step length is a value obtained by multiplying a value obtained by integrating the gyro data from the peak start point to the peak end point (corresponding to an angle difference of the foot from toe takeoff to heel landing) by a predetermined coefficient.

The analysis unit 16 outputs the extracted gait features as an analysis result.

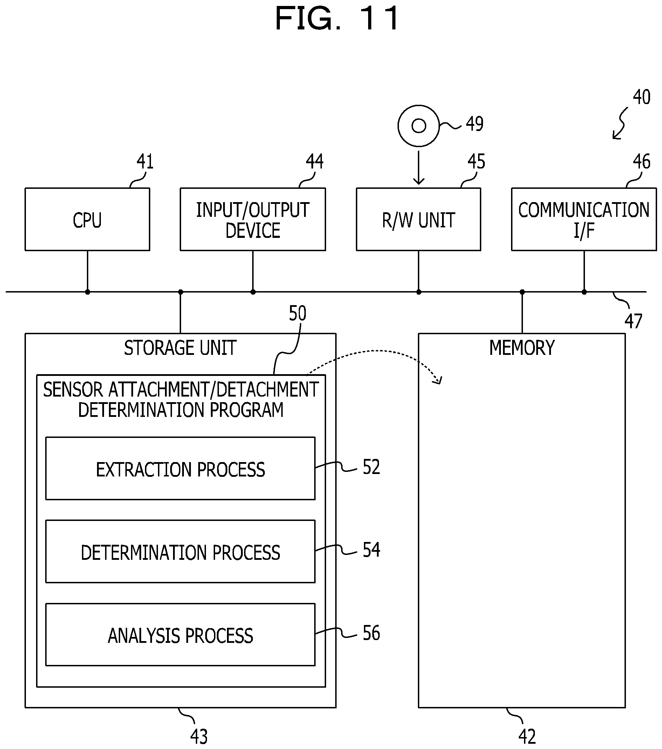

The sensor attachment/detachment determination system 10 can be implemented by, for example, a computer 40 illustrated in FIG. 11. The computer 40 includes a central processing unit (CPU) 41, a memory 42 as a temporary storage region, and a nonvolatile storage unit 43. Furthermore, the computer 40 also includes an input/output device 44 such as an input unit or a display unit, a read/write (R/W) unit 45 that controls reading and writing of data to and from a storage medium 49. Furthermore, the computer 40 includes a communication I/F 46 connected to a network such as the Internet. The CPU 41, the memory 42, the storage unit 43, the input/output device 44, the R/W unit 45, and the communication I/F 46 are connected to each other via a bus 47.

The storage unit 43 can be implemented by a hard disk drive (HDD), a solid state drive (SSD), a flash memory, or the like. The storage unit 43 as a storage medium stores a sensor attachment/detachment determination program 50 for causing the computer 40 to function as the sensor attachment/detachment determination system 10. The sensor attachment/detachment determination program 50 includes an extraction process 52, a determination process 54, and an analysis process 56.

The CPU 41 reads out the sensor attachment/detachment determination program 50 from the storage unit 43, expands the sensor attachment/detachment determination program 50 in the memory 42, and sequentially executes the processes included in the sensor attachment/detachment determination program 50. The CPU 41 executes the extraction process 52 so as to operate as the extraction unit 12 illustrated in FIG. 2. Furthermore, the CPU 41 executes the determination process 54 to operate as the determination unit 14 illustrated in FIG. 2. The CPU 41 executes the analysis process 56 so as to operate as the analysis unit 16 illustrated in FIG. 2. Thereby, the computer 40 that has executed the sensor attachment/detachment determination program 50 functions as the sensor attachment/detachment determination system 10. Note that the CPU 41 that executes programs is hardware.

Note that functions implemented by the sensor attachment/detachment determination program 50 can also be implemented by, for example, a semiconductor integrated circuit, more specifically, an application specific integrated circuit (ASIC) or the like.

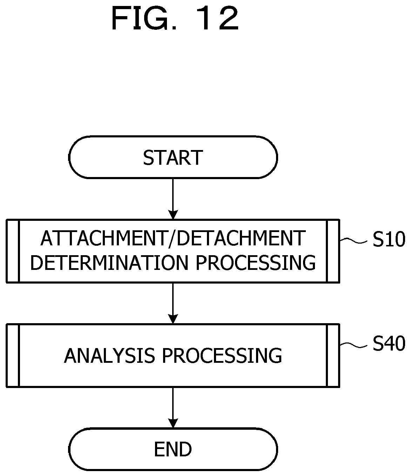

Next, an operation of the sensor attachment/detachment determination system 10 according to the first embodiment will be described. When the gyro data is input to the sensor attachment/detachment determination system 10, the sensor attachment/detachment determination system 10 executes sensor attachment/detachment determination processing illustrated in FIG. 12. Note that the sensor attachment/detachment determination processing is an example of the sensor attachment/detachment determination method of the disclosed technology.

In step S10, attachment/detachment determination processing to be described below is executed, and then in step S40, analysis processing to be described below is executed.

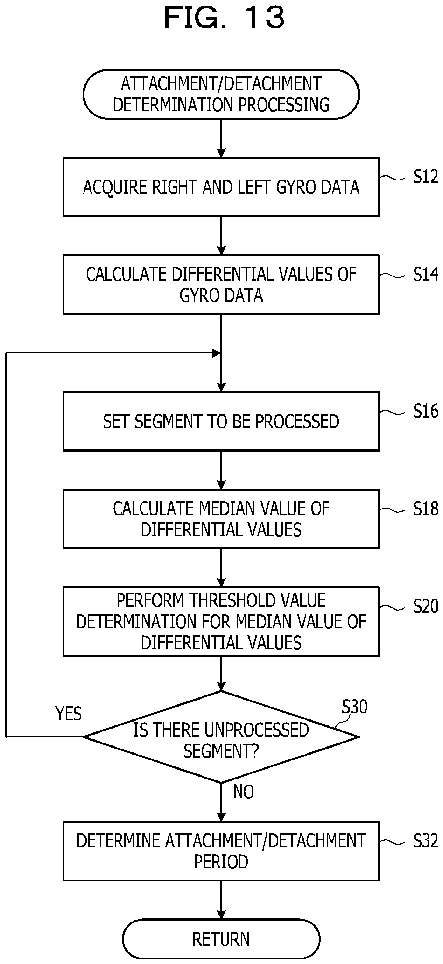

Here, the attachment/detachment determination processing will be described in detail with reference to FIG. 13.

In step S12, the extraction unit 12 acquires the gyro data output from each of the left and right gyro sensors.

Next, in step S14, the extraction unit 12 calculates the differential value ($d(t) = x(t) - x(t - 1)$) of the value at each time of the acquired gyro data.

Next, in step S16, the extraction unit 12 divides the gyro data, which is time-series data, into segments in predetermined time unit, and sets one segment as a segment to be processed in chronological order.

Next, in step S18, the extraction unit 12 calculates the median value of the differential values in the segment to be processed. The extraction unit 12 passes the extraction result in which the calculated median value of the differential values and the segment number of the segment to be processed are associated with each other to the determination unit 14.

Next, in step S20, the determination unit 14 determines whether the median value of the differential values is equal to or greater than the threshold value TH1 on the basis of the extraction result passed from the extraction unit 12, and obtains the threshold value determination result. Specifically, the determination unit 14 assigns the flag "1" to the segment in which the median value of the differential values is equal to or larger than the threshold value TH1, and assigns the flag "0" to the segment in which the median value of the differential values is less than the threshold value TH1, as the threshold value determination result.

Next, in step S30, the extraction unit 12 determines whether there is a segment for which the processing in steps S18 and S20 described above has not been processed in the gyro data. In a case where there is the unprocessed segment, the processing returns to step S16, and the extraction unit 12 sets the next segment as the segment to be processed. On the other hand, in a case where processing for all the segments is completed, the processing moves onto step S32.

In step S32, in the case where the time between successive segments in chronological order is equal to or less than a predetermined time, among the segments to which the flag "1" is assigned of the threshold value determination result, the determination unit 14 changes the flag of a segment between the segments to "1". Thereby, the threshold value determination results are connected. Then, the determination unit 14 determines a section obtained by adding a predetermined margin to areas before and after the sections of the connected threshold value determination results as the attachment/detachment period of the sensor. The determination unit 14 specifies the attachment/detachment period in which the attachment/detachment period determined in the segment unit is caused to correspond to the time of the gyro data, which is the original time-series data, and passes information of the specified attachment/detachment period to the analysis unit 16. Then, the processing returns to the sensor attachment/detachment determination processing illustrated in FIG. 12.

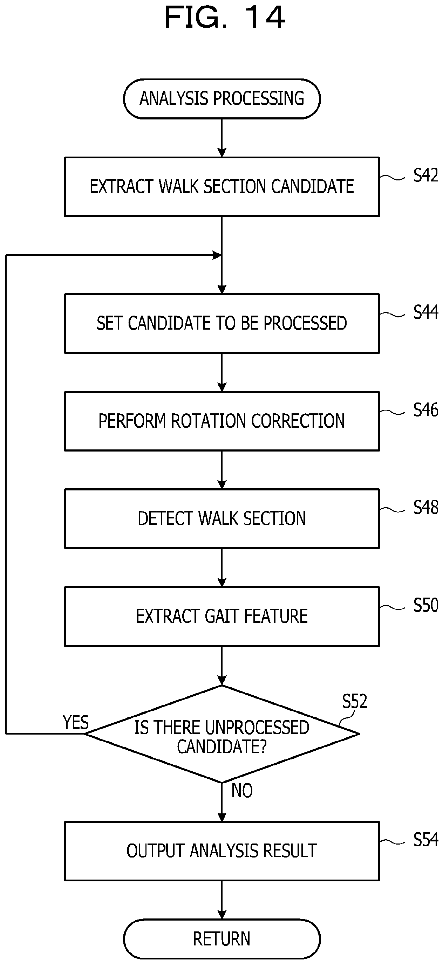

Next, the analysis processing will be described in detail with reference to FIG. 14.

In step S42, the analysis unit 16 sets the data of the sections obtained by excluding the data in the attachment/detachment period passed from the determination unit 14, of the input gyro data, as the data to be analyzed. Then, the analysis unit 16 sets while shifting a predetermined window width (for example, five seconds) for the data to be analyzed excluding the attachment/detachment period, and extracts a section having a change in the value of data in the window width as a walk section candidate.

Next, in step S44, the analysis unit 16 sets one of the walk section candidates as a candidate to be processed.

Next, in step S46, the analysis unit 16 performs processing of the rotation operation, filtering, peak detection, evaluation, and selection, as the rotation correction for the gyro data that is the set candidate.

Next, in step S48, the analysis unit 16 performs the peak range determination, condition determination element extraction, and walk condition determination as the detection of the walk section, and determines whether the candidate to be processed is a walk section.

Next, in step S50, the analysis unit 16 extracts the swing time, stride time, step length, and the like for each step included in the walk section as the gait features from the gyro data detected as the walk section in step S48 described above. The analysis unit 16 records the extracted gait features as an analysis result. Note that, in step S48 described above, in a case where the candidate to be processed is not determined as a walk section, the processing of the present step is skipped.

Next, in step S52, the analysis unit 16 determines whether there is a candidate for which the processing in steps S46 to S50 described above has not been processed, among the walk section candidates extracted in step S42 described above. In a case where there is an unprocessed candidate, the processing returns to step S44, and the analysis unit 16 sets the next candidate as the candidate to be processed. On the other hand, in a case where processing for all the candidates is completed, the processing moves onto step S54.

In step S54, the analysis unit 16 outputs the analysis result recorded in step S50 described above, and the analysis processing and the sensor attachment/detachment determination processing (FIG. 12) are completed.

As described above, according to the sensor attachment/detachment determination system of the first embodiment, the attachment/detachment period of the sensor is determined according to the section in which data indicating a high-frequency characteristic in which the frequency is equal to or higher than a predetermined value is continuously generated, among the gyro data that is time-series data. Thereby, the data at the time of attachment/detachment of the sensor, which is not data to be analyzed, can be accurately determined.

Furthermore, by excluding the data in the attachment/detachment period of the sensor determined with high accuracy from the data to be analyzed, an appropriate analysis result can be obtained as compared with a case of not excluding the data in the attachment/detachment period.

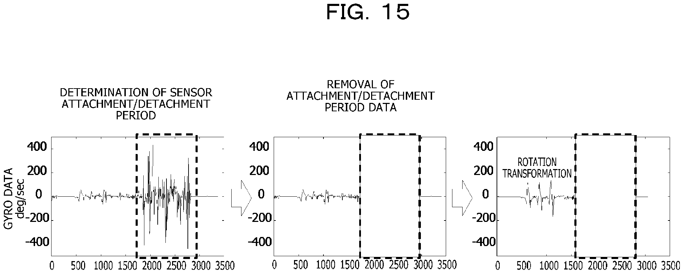

For example, as illustrated in FIG. 15, the attachment/detachment period is removed from the gyro data, and then, a rotation matrix is obtained, rotation transformation is performed by multiplying the rotation matrix, and the detection of the walk section and the extraction of the gait features are performed. In this case, inclusion of a peak generated at the time of attachment/detachment of the sensor as the peak of the waveform of the gyro data used in performing the rotation transformation and extracting the gait feature can be avoided. Thereby, effects of improvement of the correction accuracy for the mount deviation of the sensor or the like, reduction of erroneous detection of the walk section, improvement of the extraction accuracy of the gait feature, and the like can be exerted.

<Second Embodiment

Next, a second embodiment will be described. Note that, in a sensor attachment/detachment determination system according to the second embodiment, similar parts to those of the sensor attachment/detachment determination system 10 according to the first embodiment are designated by the same reference numerals and detailed description thereof will be omitted.

In the first embodiment, as a characteristic of the gyro data at the time of attachment/detachment of the sensor, a case where data indicating a high-frequency characteristic is continuously generated has been described, whereas in the second embodiment, a characteristic at the time of comparing right and left gyro data is also used in addition to the above characteristic.

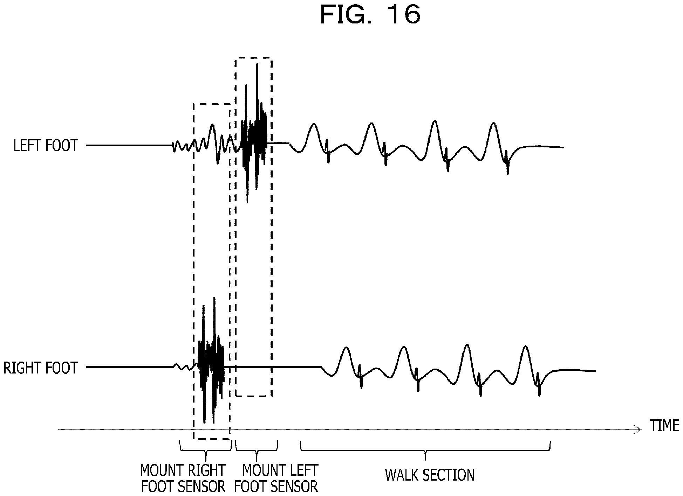

Specifically, in a case where sensors are alternately attached/detached to/from the right and left feet, as illustrated in FIG. 16, a right-left difference occurs at timing when a high-frequency characteristic is generated at the time of attachment/detachment of the sensors. This is because when the sensor is attached/detached to/from one foot, the sensor on another foot side is already attached and fixed or is left unattached. Furthermore, as illustrated in FIG. 16, a foot belonging side where the high-frequency characteristic is generated is reversed right and left during attachment/detachment of the sensor. This is because attachment/detachment of the sensor is alternately performed right and left.

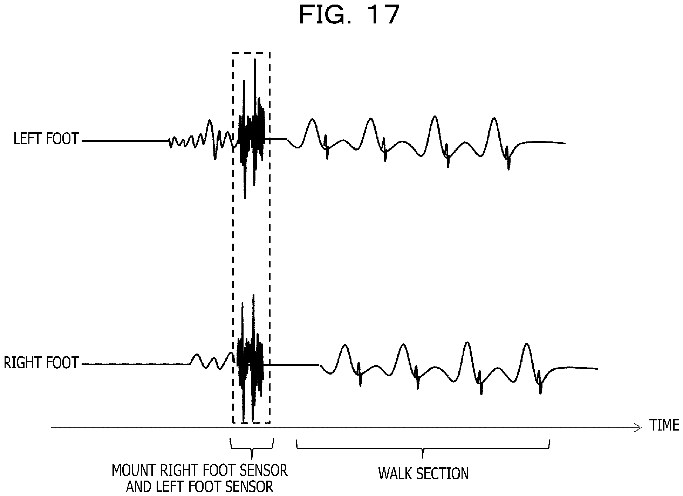

Furthermore, in a case where the sensors for both feet are attached/detached at the same time with a help of another person or the like, as illustrated in FIG. 17, there is almost no right-left difference in the timing when the high-frequency characteristic occurs.

In the second embodiment, an attachment/detachment period of a sensor is determined using the above-described characteristic as well.

The sensor attachment/detachment determination system 210 according to the second embodiment functionally includes an extraction unit 212, a determination unit 214, and an analysis unit 16 as illustrated in FIG. 2.

Similar to the extraction unit 12 in the first embodiment, the extraction unit 212 calculates a differential value of each of right and left gyro data, and obtains a median value of the differential values for each segment.

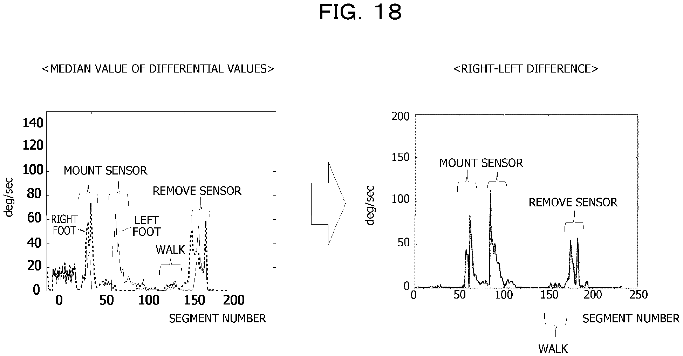

Furthermore, as illustrated in FIG. 18, the extraction unit 212 calculates an absolute value (hereinafter referred to as "right-left difference") a(n) that is a difference between the median value of the differential values of the left gyro data and the median value of the differential values of the right gyro data for each segment according to the following arithmetic equation.

$$\alpha(n) = |d_L(n) - d_R(n)|$$

Note that $d_L(n)$ is the median value of the differential values in a segment n of the left gyro data, and $d_R(n)$ is the median value of the differential values in the segment n of the right gyro data.

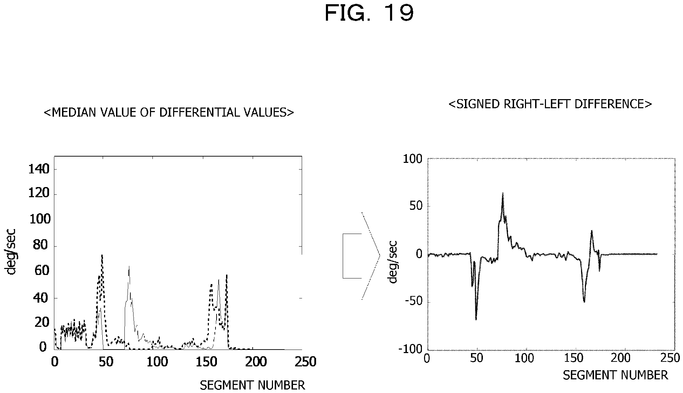

Furthermore, as illustrated in FIG. 19, the extraction unit 212 calculates a signed value (hereinafter referred to as "signed right-left difference") $\beta(n)$ between the median value of the differential values of the left gyro data and the median value of the differential values of the right gyro data for each segment according to the following arithmetic equation.

$$\beta(n) = d_L(n) - d_R(n)$$

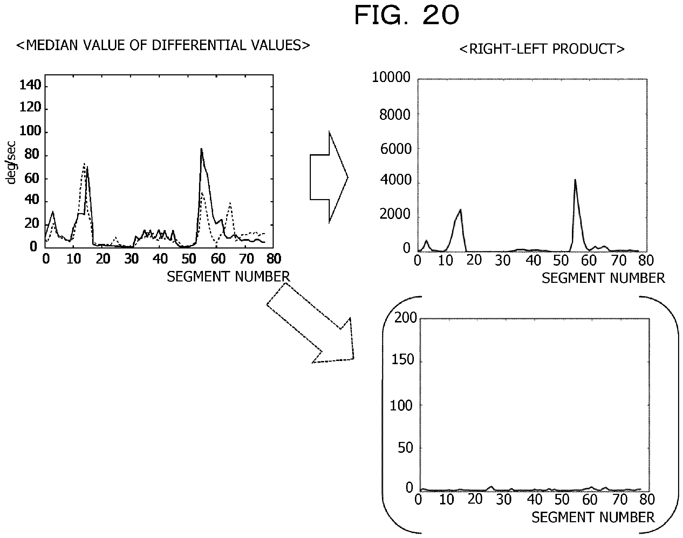

Furthermore, as illustrated in FIG. 20, the extraction unit 212 calculates a product (hereinafter referred to as "right-left product") $\gamma(n)$ of the median value of the differential values of the left gyro data and the median value of the differential values of the right gyro data for each segment according to the following arithmetic equation.

$$\gamma(n)=d_L(n)\times d_R(n)$$

Note that, in the case of attaching/detaching the right and left sensors at the same time, no remarkable characteristic is observed in the right-left difference, as illustrated in the lower right figure of FIG. 20.

The extraction unit 212 passes an extraction result in which a segment number is associated with the right-left difference, signed right-left difference, and right-left product calculated as described above to the determination unit 14.

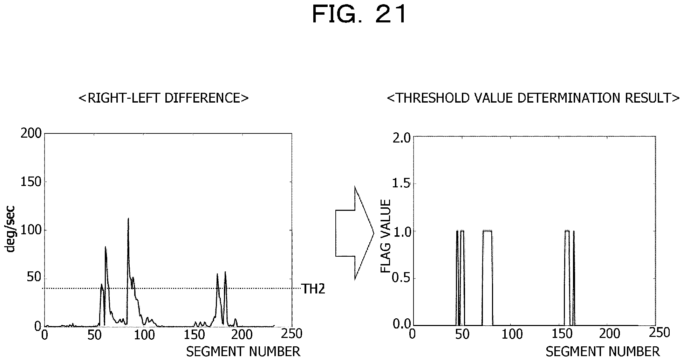

As illustrated in FIG. 21, the determination unit 214 specifies data having the right-left difference that is equal to or larger than a predetermined threshold value TH2 as the data indicating a high-frequency characteristic at the time of attachment/detachment of the sensors. Specifically, the determination unit 214 assigns a flag "1" to a segment having the right-left difference that is equal to or larger than the threshold value TH2, and assigns a flag "0" to a segment having the right-left difference that is less than the threshold value TH2.

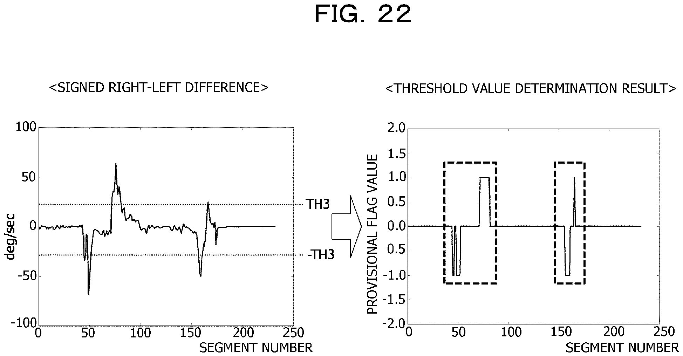

Furthermore, as illustrated in FIG. 22, the determination unit 214 specifies segment data in which a segment having the signed right-left difference that is equal to or larger than a predetermined threshold value TH3, and having the signed right-left difference that becomes equal to or less than a threshold value -TH3 within a predetermined time before and after the segment is present, as the data indicating a high-frequency characteristic. Similarly, the determination unit 214 specifies segment data in which a segment having the signed right-left difference that is equal to or less than the predetermined threshold value -TH3, and having the signed right-left difference that becomes equal to or larger than the threshold value TH3 within a predetermined time before and after the segment is present, as the data indicating a high-frequency characteristic.

Specifically, as illustrated in FIG. 22, the determination unit 214 assigns a provisional flag "1" to the segment having the signed right-left difference of the threshold value TH3 or larger, a provisional flag "-1" to the segment having the signed right-left difference of the threshold value -TH3 or less, and a flag "0" to the segment having the signed right-left difference from - TH3 to TH3, both exclusive. Then, the determination unit 214 assigns a flag indicating a final threshold value determination result to the each segment to which the provisional flag "1" or "-1" is assigned. More specifically, in a case where a segment to which a provisional flag with an opposite sign to a segment concerned is assigned is present among segments of a predetermined time before and after the segment concerned, the determination unit 214 adopts the provisional flag "1" of the segment concerned. In a case where no segment to which the provisional flag with an opposite sign is assigned is present, the determination unit 214 changes the provisional flag of the segment concerned to the flag "0".

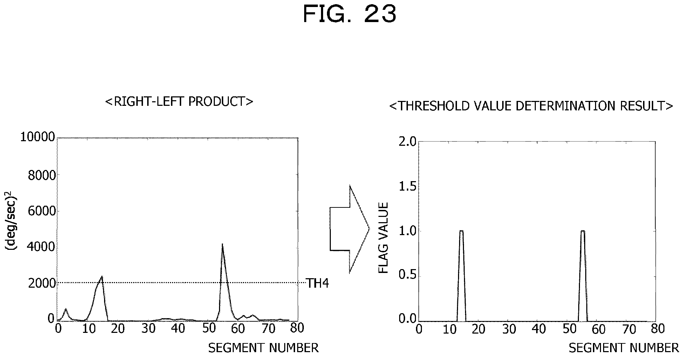

Furthermore, as illustrated in FIG. 23, the determination unit 214 specifies data having the right-left product that is equal to or larger than a predetermined threshold value TH4 as the data indicating a high-frequency characteristic at the time of attachment/detachment of the sensors. Specifically, the determination unit 214 assigns a flag "1" to a segment having the right-left product that is equal to or larger than the threshold value TH4, and assigns a flag "0" to a segment having the right-left difference that is less than the threshold value TH4.

The determination unit 214 integrates the threshold value determination results based on the right-left difference, the signed right-left difference, and the right-left product, respectively, to specify the final threshold value determination result. Specifically, the determination unit 214 assigns the final flag "1" to the segment in the case where both the flags based on the right-left difference and the signed right-left difference are "1", or in the case where the flag based on the right-left product is "1". Meanwhile, the determination unit 214 assigns the final flag "0" to the segment in the case where either one of the flags based on the right-left product and the signed right-left difference is "0" and the flag based on the right-left product is "0". As a result, the attachment/detachment period can be appropriately determined even in a case where whether the sensors are attached/detached by one person or two persons is not known in advance.

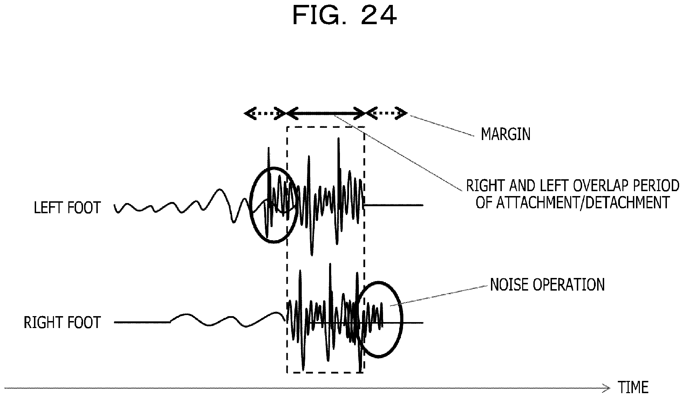

The determination unit 214 connects the segments with the final flag of "1" and adds a margin, and determines the attachment/detachment period of the sensor, similarly to the determination unit 14 in the first embodiment. At this time, for the segment with the final flag of "1" on the basis of the right-left product, the margin to be added may be set to be longer than that of the segment with the final flag of "1" on the basis of the right-left difference. As illustrated in FIG. 24, even if the sensors are attached/detached at the same time on both feet, the timings are rarely exactly the same and in most cases, some of them overlap. Therefore, the long margin is added in consideration of the fact that there is a period in which the attachment/detachment period does not overlap before and after the period where the right-left product is the threshold value TH4 or larger.

The sensor attachment/detachment determination system 210 can be implemented by, for example, a computer 40 illustrated in FIG. 11. The storage unit 43 of the computer 40 stores a sensor attachment/detachment determination program 250 for causing the computer 40 to function as the sensor attachment/detachment determination system 210. The sensor attachment/detachment determination program 250 includes an extraction process 252, a determination process 254, and an analysis process 56.

The CPU 41 reads out the sensor attachment/detachment determination program 250 from the storage unit 43, expands the sensor attachment/detachment determination program 250 in the memory 42, and sequentially executes the processes included in the sensor attachment/detachment determination program 250. The CPU 41 executes the extraction process 252 so as to operate as the extraction unit 212 illustrated in FIG. 2. Furthermore, the CPU 41 executes the determination process 254 to operate as the determination unit 214 illustrated in FIG. 2. The CPU 41 executes the analysis process 56 so as to operate as the analysis unit 16 illustrated in FIG. 2. Thereby, the computer 40 that has executed the sensor attachment/detachment determination program 250 functions as the sensor attachment/detachment determination system 210.

Note that the functions implemented by the sensor attachment/detachment determination program 250 can also be implemented by, for example, a semiconductor integrated circuit, more specifically, an ASIC or the like.

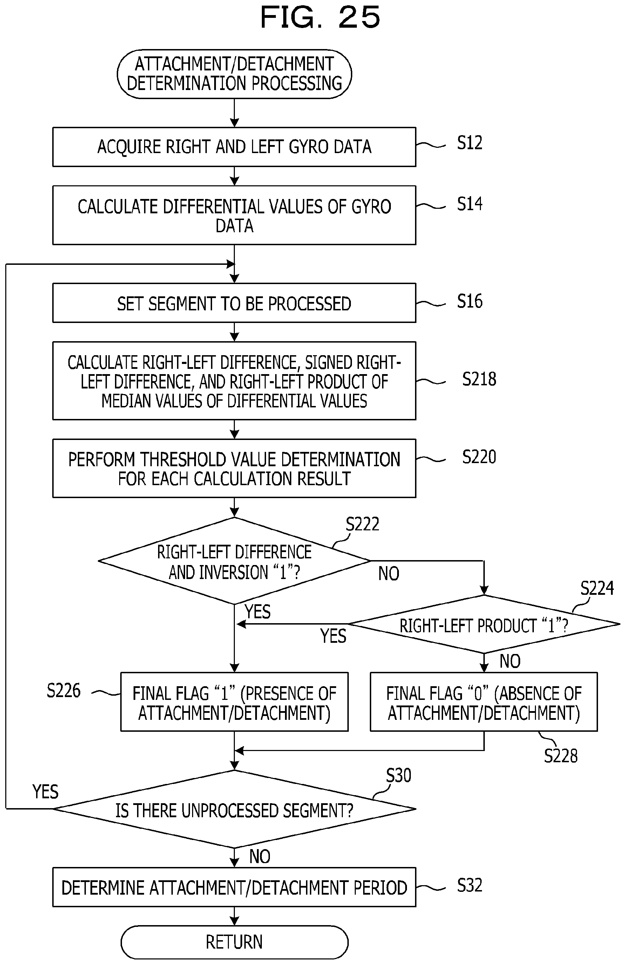

Next, an operation of the sensor attachment/detachment determination system 10 according to the second embodiment will be described. In the second embodiment, attachment/detachment determination processing executed in step S10 of sensor attachment/detachment determination processing illustrated in FIG. 12 is different from that of the first embodiment. Therefore, the attachment/detachment determination processing in the second embodiment will be described with reference to FIG. 25. Note that processing similar to the attachment/detachment determination processing (FIG. 13) in the first embodiment is designated by the same reference numerals and detailed description thereof will be omitted.

After steps S12 to S16, in next step S218, the extraction unit 212 calculates the right-left difference, the signed right-left difference, and the right-left product of the median values of the differential values of the gyro data in the segment to be processed.

Next, in step S220, the determination unit 214 performs threshold value determination on the basis of the respective calculation result of the right-left difference, the signed right-left difference, and the right-left product, and assigns the flag "1" to the segment that is the candidate for the attachment/detachment period and the flag "0" to segments other than the candidate segment.

Next, in step S222, the determination unit 214 determines whether both the flags based on the right-left difference and the signed right-left difference assigned to the segment to be processed are "1". In a case where both the flags are "1", the processing proceeds to step S226, and in a case where either one of the flags is "0", the processing proceeds to step S224.

In step S224, the determination unit 214 determines whether the flag based on the right-left product assigned to the segment to be processed is "1". In a case where the flag is "1", the processing proceeds to step S226, and in a case where the flag is "0", the processing proceeds to step S228.

In step S226, the determination unit 214 assigns the final flag "1" to the segment to be processed. Meanwhile, in step S228, the determination unit 214 assigns the final flag "0" to the segment to be processed.

Hereinafter, steps S30 and S32 are similarly executed to those in the first embodiment, and the attachment/detachment determination processing is completed.

As described above, according to the sensor attachment/detachment determination system of the second embodiment, as the characteristic of the gyro data at the time of attachment/detachment of the sensor, the characteristic at the time of comparing right and left gyro data is also used in addition to the case where data indicating a high-frequency characteristic is continuously generated. Thereby, in the case where the two gyro sensors are mounted at positions making a pair on the left and right sides of the human body, the data at the time of attachment/detachment of the sensors, which is not the data to be analyzed, can be determined with higher accuracy.

Note that, in each of the above-described embodiments, a case of acquiring the gyro data when the gyro sensors are mounted to both feet has been described, but the embodiment is not limited to the case. For example, the embodiment can be also applied to a case where the sensors are attached to parts such as wrists or arms. Furthermore, the first embodiment is not limited to the case where the two gyro sensors are mounted at the positions making a pair on the right and left sides of the human body, and can be also applied to a case where one, or three or more gyro sensors are mounted although the case depends on the content of the analysis processing. In the case where three or more gyro sensors are mounted, attachment/detachment period determination and analysis processing for each gyro sensor may be simply performed by the technique of the first embodiment, for gyro data output from each gyro sensor.

Furthermore, in each of the above-described embodiments, a case of extracting the gait feature from the gyro data has been described as the analysis processing, but the embodiment is not limited to the case. The analysis processing according to the part to which the gyro sensor is mounted and the movement of the part may be appropriately performed.

Furthermore, in the above-described second embodiment, a case of determining the sensor attachment/detachment period using all the right-left difference, the signed right-left difference, and the right-left product of the median values for each segment of the differential values of the gyro data has been described. However, the embodiment is not limited to the case. Determination may be made using any one of the right-left difference, the signed right-left difference, and the right-left product.

Furthermore, in the above-described second embodiment, a case of calculating the right-left difference, the signed right-left difference, and the right-left product after calculating the median value for each segment of the differential values of the gyro data has been described, but the embodiment is not limited to the case. First, the right-left difference, the signed right-left difference, and the right-left product of the differential values are calculated, and then the median value of the calculation results may be calculated for each segment.

Furthermore, a ratio may be used instead of the difference between the left gyro data and the right gyro data in the above-described second embodiment. Furthermore, a sum may be used instead of the product of the left gyro data and the right gyro data.

Furthermore, in each of the above-described embodiments, the case of using the differential values of the gyro data for extracting the frequency characteristic of the gyro data has been described. However, the embodiment is not limited to the case. A frequency analysis may be performed for each time, and whether the high-frequency characteristic is exhibited may be specified.

Furthermore, in each of the above-described embodiments, the case of specifying the section in which the data indicating a high-frequency characteristic is continuously generated by calculating the median value of the differential values for each segment of the gyro data and performing the threshold value determination has been described, but the embodiment is not limited to the case. For example, another method may be adopted, such as specifying a section in which the frequency of occurrence of the differential value equal to or larger than the threshold value is equal to or higher than a predetermined ratio.

Furthermore, in each of the above-described embodiments, the case where the sensor attachment/detachment determination system is implemented by one computer has been described. However, the embodiment is not limited to the case. Each functional unit may be distributed to a plurality of computers.

Furthermore, in each of the above-described embodiments, an embodiment in which the sensor attachment/detachment determination program is stored (installed) in advance in the storage unit has been described. However, the embodiment is not limited to the case. The program according to the disclosed technology can also be provided in a form stored in a storage medium such as a compact disc read only memory (CD-ROM), a digital versatile disc read only memory (DVD-ROM), or a universal serial bus (USB) memory.

CITATION LIST

PATENT DOCUMENT

Japanese Laid-open Patent Publication No. 2000-325329;
Japanese Laid-open Patent Publication No. 2012-343;
Japanese Laid-open Patent Publication No. 2014-196941; and
International Publication Pamphlet No. WO 2018/092219.

REFERENCE SIGNS LIST

10, 210 Sensor attachment/detachment determination system
12, 212 Extraction unit
14, 214 Determination unit
16 Analysis unit
40 Computer
41 CPU
42 Memory
43 Storage unit
49 Storage medium
50, 250 Sensor attachment/detachment determination program

**Claims**

1. A sensor attachment/detachment determination program comprising instructions which, when the program is executed by a computer, cause the computer to execute processing, the processing comprising:

   extracting a frequency characteristic of time-series data acquired from a gyro sensor; and
   determining a sensor attachment/detachment period according to a section in which data indicating a high-frequency characteristic in which a frequency is equal to or higher than a predetermined value is continuously generated, among the time-series data, on the basis of the frequency characteristic.

2. The sensor attachment/detachment determination program according to claim 1, wherein, in a case where a statistical value of a value indicating the frequency characteristic included in a predetermined time unit is equal to or larger than a predetermined first threshold value, a section in the predetermined time unit is determined as the section in which data indicating a high-frequency characteristic is continuously generated.

3. The sensor attachment/detachment determination program according to claim 1 or 2, wherein,

in a case where the two gyro sensors are mounted at positions making a pair on right and left of a human body, a difference or a ratio between a value indicating the frequency characteristic of the time-series data acquired from the left gyro sensor and a value indicating the frequency characteristic of the time-series data acquired from the right gyro sensor is extracted as the value indicating the frequency characteristic, and data in which the difference or the ratio falls within a predetermined first predetermined range is specified as the data indicating a high-frequency characteristic.

4. The sensor attachment/detachment determination program according to any one of claims 1 to 3, wherein,

in a case where the two gyro sensors are mounted at positions making a pair on right and left of a human body, a difference or a ratio between a value indicating the frequency characteristic of the time-series data acquired from the left gyro sensor and a value indicating the frequency characteristic of the time-series data acquired from the right gyro sensor is extracted as the value indicating the frequency characteristic, and first data in which second data having the difference or the ratio falling within a third predetermined range, of the first data having the difference or the ratio falling within a predetermined second predetermined range, is generated within a predetermined time including the first data, and the first data in which the second data having the difference or the ratio falling within the second predetermined range, of the first data having the difference or the ratio falling within a predetermined third predetermined range, is generated within the predetermined time including the first data, are specified as the data indicating a high-frequency characteristic.

5. The sensor attachment/detachment determination program according to any one of claims 1 to 4, wherein,

in a case where the two gyro sensors are mounted at positions making a pair on right and left of a human body, a product or a sum of a value indicating the frequency characteristic of the time-series data acquired from the left gyro sensor and a value indicating the frequency characteristic of the time-series data acquired from the right gyro sensor is extracted as the value indicating the frequency characteristic, and data in which the product or the sum becomes equal to or larger than a predetermined second threshold value is specified as the data indicating a high-frequency characteristic.

6. The sensor attachment/detachment determination program according to any one of claims 1 to 5, wherein, in a case where an interval of successive sections among a plurality of sections in which the data indicating a high-frequency characteristic is continuously generated is equal to or smaller than a predetermined time, a section in which the successive sections are connected is determined as the sensor attachment/detachment period.

7. The sensor attachment/detachment determination program according to any one of claims 1 to 6, wherein a section obtained by adding a predetermined margin to areas before and after a section in which the data indicating a high-frequency characteristic is continuously generated is determined as the sensor attachment/detachment period.

8. A sensor attachment/detachment determination system comprising:

an extraction unit configured to extract a frequency characteristic of time-series data acquired from a gyro sensor; and
a determination unit configured to determine a sensor attachment/detachment period according to a section in which data indicating a high-frequency characteristic in which a frequency is equal to or higher than a predetermined value is continuously generated, among the time-series data, on the basis of the frequency characteristic.

9. The sensor attachment/detachment determination system according to claim 8, wherein, in a case where a statistical value of a value indicating the frequency characteristic included in a predetermined time unit is equal to or larger than a predetermined first threshold value, the determination unit determines a section in the predetermined time unit as the section in which data indicating a high-frequency characteristic is continuously generated.

10. The sensor attachment/detachment determination system according to claim 8 or 9, wherein,

in a case where the two gyro sensors are mounted at positions making a pair on right and left of a human body, the extraction unit extracts a difference or a ratio between a value indicating the frequency characteristic of the time-series data acquired from the left gyro sensor and a value indicating the frequency characteristic of the time-series data acquired from the right gyro sensor as the value indicating the frequency characteristic, and the determination unit specifies data in which the difference or the ratio falls within a predetermined first predetermined range as the data indicating a high-frequency characteristic.

11. The sensor attachment/detachment determination system according to any one of claims 8 to 10, wherein,

in a case where the two gyro sensors are mounted at positions making a pair on right and left of a human body, the extraction unit extracts a difference or a ratio between a value indicating the frequency characteristic of the time-series data acquired from the left gyro sensor and a value indicating the frequency characteristic of the time-series data acquired from the right gyro sensor as the value indicating the frequency characteristic, and the determination unit specifies first data in which second data having the difference or the ratio falling within a third predetermined range, of the first data having the difference or the ratio falling within a predetermined second predetermined range, is generated within a predetermined time including the first data, and the first data in which the second data having the difference or the ratio falling within the second predetermined range, of the first data having the difference or the ratio falling within a predetermined third predetermined range, is generated within the predetermined time including the first data, as the data indicating a high-frequency characteristic.

12. The sensor attachment/detachment determination system according to any one of claims 8 to 11, wherein,

in a case where the two gyro sensors are mounted at positions making a pair on right and left of a human body, the extraction unit extracts a product or a sum of a value indicating the frequency characteristic of the time-series data acquired from the left gyro sensor and a value indicating the frequency characteristic of the time-series data acquired from the right gyro sensor as the value indicating the frequency characteristic, and the determination unit specifies data in which the product or the sum becomes equal to or larger than a predetermined second threshold value as the data indicating a high-frequency characteristic.

13. The sensor attachment/detachment determination system according to any one of claims 8 to 12, wherein, in a case where an interval of successive sections among a plurality of sections in which the data indicating a high-frequency characteristic is continuously generated is equal to or smaller than a predetermined time, the determination unit determines a section in which the successive sections are connected as the sensor attachment/detachment period.

14. The sensor attachment/detachment determination system according to any one of claims 8 to 13, wherein the determination unit determines a section obtained by adding a predetermined margin to areas before and after a section in which the data indicating a high-frequency characteristic is continuously generated as the sensor attachment/detachment period.

15. A method of a sensor attachment/detachment determination, the method comprising:

extracting a frequency characteristic of time-series data acquired from a gyro sensor; and
determining a sensor attachment/detachment period according to a section in which data indicating a high-frequency characteristic in which a frequency is equal to or higher than a predetermined value is continuously generated, among the time-series data, on the basis of the frequency characteristic.

16. The method according to claim 15, wherein, in a case where a statistical value of a value indicating the frequency characteristic included in a predetermined time unit is equal to or larger than a predetermined first threshold value, a section in the predetermined time unit is determined as the section in which data indicating a high-frequency characteristic is continuously generated.

# FIG. 1

# FIG. 2

GYRO DATA

10(210)

12(212)

EXTRACTION UNIT

14(214)

DETERMINATION UNIT

16

ANALYSIS UNIT

ANALYSIS RESULT

# FIG. 3

<GYRO DATA>   <DIFFERENTIAL VALUE (EACH TIME)>   <MEDIAN VALUE OF DIFFERENTIAL VALUES>

# FIG. 4

<MEDIAN VALUE OF DIFFERENTIAL VALUES>

<THRESHOLD VALUEDETERMINATION RESULT>

# FIG. 5

<THRESHOLD VALUEDETERMINATION RESULT>

<ATTACHMENT/DETACHMENT PERIOD DETERMINATION RESULT>

# FIG. 6

<ATTACHMENT/DETACHMENT PERIOD
DETERMINATION RESULT>

FIG. 7

FIG. 8

EVALUATION VALUE
= NONE

EVALUATION VALUE
= 340

EVALUATION VALUE
= 125

| ROTATION OPERATION | FILTERING | PEAK DETECTION | EVALUATION AND SELECTION |

# FIG. 9

# FIG. 10

| STEP | FIRST STEP | SECOND STE | THIRD STEP | FOURTH STEP | FIFTH STEP | SIXTH STEP | SEVENTH STEP | EIGHTH STEP | CONDITION DETERMINATION |
|---|---|---|---|---|---|---|---|---|---|
| PEAK INTERVAL | 1.98 | 1.75 | 1.54 | 1.66 | 1.62 | 1.60 | 1.81 | --- | O |
| PEAK AMPLITUDE | 321 | 354 | 367 | 355 | 329 | 352 | 344 | 319 | O |
| LANDING TIME | 0.087 | 0.078 | 0.081 | 0.075 | 0.082 | 0.081 | 0.079 | --- | O |
| BELONGING SIDE | LEFT | RIGHT | LEFT | RIGHT | LEFT | RIGHT | LEFT | RIGHT | O |

# FIG. 11

| | | | |
|---|---|---|---|
| CPU <br> 41 | INPUT/OUTPUT DEVICE <br> 44 | R/W UNIT <br> 45 | COMMUNICATION I/F <br> 46 |

49

40

47

**STORAGE UNIT** 43

50

**SENSOR ATTACHMENT/DETACHMENT DETERMINATION PROGRAM**

EXTRACTION PROCESS — 52

DETERMINATION PROCESS — 54

ANALYSIS PROCESS — 56

**MEMORY** 42

# FIG. 12

```
        ┌─────────────────┐
        │      START       │
        └─────────────────┘
                 │
                 ▼
  ┌──────────────────────────────┐
  │  ATTACHMENT/DETACHMENT        │ ～ S10
  │  DETERMINATION PROCESSING     │
  └──────────────────────────────┘
                 │
                 ▼
  ┌──────────────────────────────┐
  │    ANALYSIS PROCESSING        │ ～ S40
  └──────────────────────────────┘
                 │
                 ▼
        ┌─────────────────┐
        │       END        │
        └─────────────────┘
```

# FIG. 13

```
      ┌─────────────────────────────┐
      │   ATTACHMENT/DETACHMENT     │
      │   DETERMINATION PROCESSING  │
      └─────────────────────────────┘
                    │
                    ▼
      ┌─────────────────────────────┐
      │ ACQUIRE RIGHT AND LEFT      │──── S12
      │ GYRO DATA                   │
      └─────────────────────────────┘
                    │
                    ▼
      ┌─────────────────────────────┐
      │ CALCULATE DIFFERENTIAL      │──── S14
      │ VALUES OF GYRO DATA         │
      └─────────────────────────────┘
                    │
                    ▼
      ┌─────────────────────────────┐
      │ SET SEGMENT TO BE PROCESSED │──── S16
      └─────────────────────────────┘
                    │
                    ▼
      ┌─────────────────────────────┐
      │ CALCULATE MEDIAN VALUE OF   │──── S18
      │ DIFFERENTIAL VALUES         │
      └─────────────────────────────┘
                    │
                    ▼
      ┌─────────────────────────────┐
      │ PERFORM THRESHOLD VALUE     │──── S20
      │ DETERMINATION FOR MEDIAN    │
      │ VALUE OF DIFFERENTIAL VALUES│
      └─────────────────────────────┘
                    │
                    ▼
  YES            ╱────────╲          S30
 ◄─────────────  IS THERE UNPROCESSED
                  SEGMENT?
                 ╲────────╱
                    │ NO
                    ▼
      ┌─────────────────────────────┐
      │ DETERMINE ATTACHMENT/       │──── S32
      │ DETACHMENT PERIOD           │
      └─────────────────────────────┘
                    │
                    ▼
             ┌──────────────┐
             │    RETURN    │
             └──────────────┘
```

# FIG. 14

```
        ( ANALYSIS PROCESSING )
                  │
                  ▼
    ┌──────────────────────────────┐
    │  EXTRACT WALK SECTION CANDIDATE │ ─── S42
    └──────────────────────────────┘
                  │
    ┌──►          ▼
    │   ┌──────────────────────────────┐
    │   │   SET CANDIDATE TO BE PROCESSED │ ─── S44
    │   └──────────────────────────────┘
    │             │
    │             ▼
    │   ┌──────────────────────────────┐
    │   │   PERFORM ROTATION CORRECTION   │ ─── S46
    │   └──────────────────────────────┘
    │             │
    │             ▼
    │   ┌──────────────────────────────┐
    │   │      DETECT WALK SECTION        │ ─── S48
    │   └──────────────────────────────┘
    │             │
    │             ▼
    │   ┌──────────────────────────────┐
    │   │       EXTRACT GAIT FEATURE      │ ─── S50
    │   └──────────────────────────────┘
    │             │
    │             ▼                         S52
    │       ◇ IS THERE UNPROCESSED ◇
    └── YES ◇      CANDIDATE?       ◇
            ◇                       ◇
                  │ NO
                  ▼
    ┌──────────────────────────────┐
    │      OUTPUT ANALYSIS RESULT     │ ─── S54
    └──────────────────────────────┘
                  │
                  ▼
            ( RETURN )
```

# FIG. 15

# FIG. 16

LEFT FOOT

RIGHT FOOT

TIME

MOUNT RIGHT
FOOT SENSOR

MOUNT LEFT
FOOT SENSOR

WALK SECTION

# FIG. 17

LEFT FOOT

RIGHT FOOT

TIME

MOUNT RIGHT FOOT SENSOR
AND LEFT FOOT SENSOR

WALK SECTION

# FIG. 18

<MEDIAN VALUE OF DIFFERENTIAL VALUES>

<RIGHT-LEFT DIFFERENCE>

FIG. 19

<MEDIAN VALUE OF DIFFERENTIAL VALUES>

<SIGNED RIGHT-LEFT DIFFERENCE>

# FIG. 20

<MEDIAN VALUE OF DIFFERENTIAL VALUES>

<RIGHT-LEFT PRODUCT>

FIG. 21

<RIGHT-LEFT DIFFERENCE>

<THRESHOLD VALUE DETERMINATION RESULT>

# FIG. 22

<SIGNED RIGHT-LEFT DIFFERENCE>

<THRESHOLD VALUE DETERMINATION RESULT>

# FIG. 23

<RIGHT-LEFT PRODUCT>

<THRESHOLD VALUE DETERMINATION RESULT>

# FIG. 24

MARGIN

RIGHT AND LEFT OVERLAP PERIOD
OF ATTACHMENT/DETACHMENT

LEFT FOOT

NOISE OPERATION

RIGHT FOOT

TIME

# FIG. 25

```
        ┌─────────────────────────────┐
        │   ATTACHMENT/DETACHMENT      │
        │  DETERMINATION PROCESSING    │
        └─────────────────────────────┘
                      │
                      ▼
        ┌─────────────────────────────┐
        │ ACQUIRE RIGHT AND LEFT GYRO  │─── S12
        │           DATA               │
        └─────────────────────────────┘
                      │
                      ▼
        ┌─────────────────────────────┐
        │ CALCULATE DIFFERENTIAL       │─── S14
        │ VALUES OF GYRO DATA          │
        └─────────────────────────────┘
                      │
                      ▼
        ┌─────────────────────────────┐
        │  SET SEGMENT TO BE PROCESSED │─── S16
        └─────────────────────────────┘
                      │
                      ▼
        ┌─────────────────────────────┐
        │ CALCULATE RIGHT-LEFT         │
        │ DIFFERENCE, SIGNED RIGHT-    │─── S218
        │ LEFT DIFFERENCE, AND RIGHT-  │
        │ LEFT PRODUCT OF MEDIAN       │
        │ VALUES OF DIFFERENTIAL VALUES│
        └─────────────────────────────┘
                      │
                      ▼
        ┌─────────────────────────────┐
        │ PERFORM THRESHOLD VALUE      │─── S220
        │ DETERMINATION FOR EACH       │
        │ CALCULATION RESULT           │
        └─────────────────────────────┘
```

S222 — RIGHT-LEFT DIFFERENCE AND INVERSION "1"?  NO →

S224 — RIGHT-LEFT PRODUCT "1"?

YES ←  YES

NO

S226 — FINAL FLAG "1" (PRESENCE OF ATTACHMENT/DETACHMENT)

FINAL FLAG "0" (ABSENCE OF ATTACHMENT/DETACHMENT) — S228

S30 — IS THERE UNPROCESSED SEGMENT?

YES

NO

DETERMINE ATTACHMENT/DETACHMENT PERIOD — S32

RETURN

## INTERNATIONAL SEARCH REPORT

| International application No. |
|---|
| PCT/JP2019/002798 |

**A. CLASSIFICATION OF SUBJECT MATTER**
Int. Cl. A61B5/11(2006.01)i

According to International Patent Classification (IPC) or to both national classification and IPC

**B. FIELDS SEARCHED**

Minimum documentation searched (classification system followed by classification symbols)
Int. Cl. A61B5/11

Documentation searched other than minimum documentation to the extent that such documents are included in the fields searched
```
Published examined utility model applications of Japan     1922-1996
Published unexamined utility model applications of Japan   1971-2019
Registered utility model specifications of Japan           1996-2019
Published registered utility model applications of Japan   1994-2019
```

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2010-178982 A (OMRON HEALTHCARE CO., LTD.) 19 August 2010, paragraphs [0061]-[0068], fig. 7 & US | 1-2, 6-9, 13-16, 20 |
| A | 2011/0295547 A1, paragraphs [0085]-[0095], fig. 7 | 3-5, 10-12, 17-19 |
| Y | JP 2015-177925 A (NIPPON TELEGRAPH AND TELEPHONE CORP.) 08 October 2015, paragraph [0033], fig. 1-3 (Family: none) | 1-2, 6-9, 13-16, 20 |

☐ Further documents are listed in the continuation of Box C.     ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09.04.2019 | 16.04.2019 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**EP 3 918 990 A1**

**REFERENCES CITED IN THE DESCRIPTION**

**Patent documents cited in the description**

- JP 2000325329 A
- JP 2012000343 A
- JP 2014196941 A
- WO 2018092219 A